# EUROPEAN PATENT SPECIFICATION

(11) **EP 1 673 359 B1**
(45) Date of publication and mention of the grant of the patent: **09.09.2009**
(21) Application number: 04769349.4
(22) Date of filing: 13.09.2004
(51) Int. Cl.: C07D 305/12, C12P 17/02, A61K 31/365

(54) **PROCESS FOR THE PREPARATION OF CRYSTALLINE FORMS OF ORLISTAT**
VERFAHREN ZUR HERSTELLUNG KRISTALLINER FORMEN VON ORLISTAT
PROCEDE DE PREPARATION DE FORMES CRISTALLINES D'ORLISTAT

(30) Priority: 12.09.2003 IN DE11442003
(43) Date of publication of application: 28.06.2006
(73) Proprietor: Ranbaxy Laboratories Limited, Gurgaon, Haryana 122001 (IN)
(72) Inventor: KUMAR, Yatendra, Gurgaon, Haryana 122001 (IN); PRASAD, Mohan, Gurgaon, Haryana 122001 (IN); DEO, Keshav, Gurgaon, Haryana 122001 (IN); PANDEY, Anand, Pratapgarh, Uttar Pradesh 230128 (IN); PATEL, Killol, Junagadh, Gujarat 362001 (IN); KANWAR, Seema, Chandigarh, Chandigarh 160022 (IN)
(74) Representative: Cronin, Brian Harold John
(86) International application number: PCT/IB2004/002957
(87) International publication number: WO 2005/026140

(56) References cited:
- WO-A-02/098413
- WO-A-03/047531
- US-A- 6 156 911
- CAIRA M R: "CRYSTALLINE POLYMORPHISM OF ORGANIC COMPOUNDS" TOPICS IN CURRENT CHEMISTRY, SPRINGER, BERLIN, DE, vol. 198, 1998, pages 163-208, XP001156954 ISSN: 0340-1022

## Description

### Field of the Invention

The field of the invention relates to processes for preparing crystalline Form I of orlistat, which is tetrahydrolipstatin. The invention also relates to the preparation of pharmaceutical compositions that include the crystalline Forms I of orlistat.

### Background of the Invention

Orlistat is a useful pancreatic lipase-inhibiting agent and can be used for the prevention and treatment of obesity and hyperlipaemia. Chemically, it is N-formyl-L-leucine[2S-[2alpha(R*),3 beta]]-1-[(3-hexyl-4-oxo-2-oxetanyl)methyl]dodecyl ester and is known from U.S. Patent No. 4,598,089.

Several processes have been reported for the purification of orlistat by chromatographic methods using toluene and ethyl acetate for example, in U.S. Patent No. 4,983,746; Helv. Chim. Acta. 1987, 70, 1412; J. Org.Chem., 1988, 53, 1218; and 1993, 58, 7768.

WO 02/98413 describes several compositions of orlistat.

J. Chem, Soc. Perkin. Trans. 1, 1998, 17, 2679 reports the use of hexane and ethyl acetate for chromatographic purification of orlistat to obtain the product as an amorphous solid.

The importance of polymorphism in pharmaceuticals has been well documented. For example, Miro R. Caira, discuss this topic in detail the chapter "Crystalline Polymorphism of Organic Compounds" in Topics in Current Chemistry (Design of Organic Solids), Ed E. Weber, Springer, Berlin, Vol 198, 1998, pages 163-208.

U.S. Patent No. 6,156,911; Tetrahedron letters, 1989, 30, 1833; J. Org.Chem., 1991, 56, 4714; and Helv. Chim. Acta. 1987, 70, 1412 disclose a crystalline form of orlistat, obtained by recrystallization with hydrocarbons such as hexane, pentane and heptane, and characterized it by melting point and infrared spectroscopy. This crystalline form is herein after referred to as 'Form I'.

WO 03/047531 describes several processes for crystallizing orlistat Form I and Form II.

Another crystalline form of orlistat, having different X-ray diffraction pattern, is marketed by Roche as Zenica® capsules but, has not been reported in the literature. It is herein after referred to as 'Form II'.

### Summary of the Invention

According to the invention there is provided a process for the preparation of crystalline Form I of orlistat. The process includes obtaining a melt of orlistat; and drying the melt to get the Form I of orlistat.

Drying the melt may include one or more of distillation, distillation under vacuum, evaporation, and evaporation under vacuum.

In one general aspect, the melt may be cooled before drying to get a crystalline solid.

The process may include further drying of the product obtained.

The melt can be obtained by heating any known form of orlistat, including amorphous orlistat. In particular, the crystalline Form II of orlistat can be used to make the melt.

The crystalline Form II of orlistat can be made by preparing a solution of orlistat in one or more ethers; and isolating the orlistat in the crystalline Form II from the solution thereof by the removal of the ether. The ethers may include one or more of diethyl ether, diisopropyl ether, tert.-butyl-methyl ether, and tetrahydrofuran. In particular, the ether may be diisopropyl ether. Removing the ether may include one or more of distillation, distillation under vacuum, evaporation, filtration, filtration under vacuum, decantation and centrifugation. This process may include further drying of the product obtained and may produce the crystalline Form II of the orlistat having the X-ray diffraction pattern of Figure 4, the infrared spectrum of Figure 5, and the differential scanning calorimetry plot of Figure 6.

The process for forming the Form I orlistat may include further forming of the Form I orlistat product so obtained into a finished dosage form.

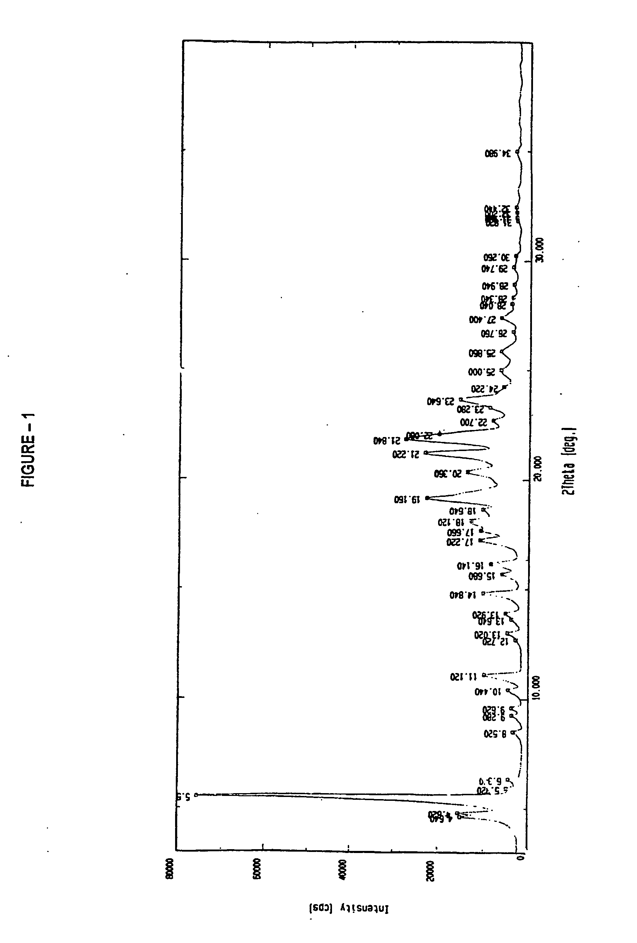

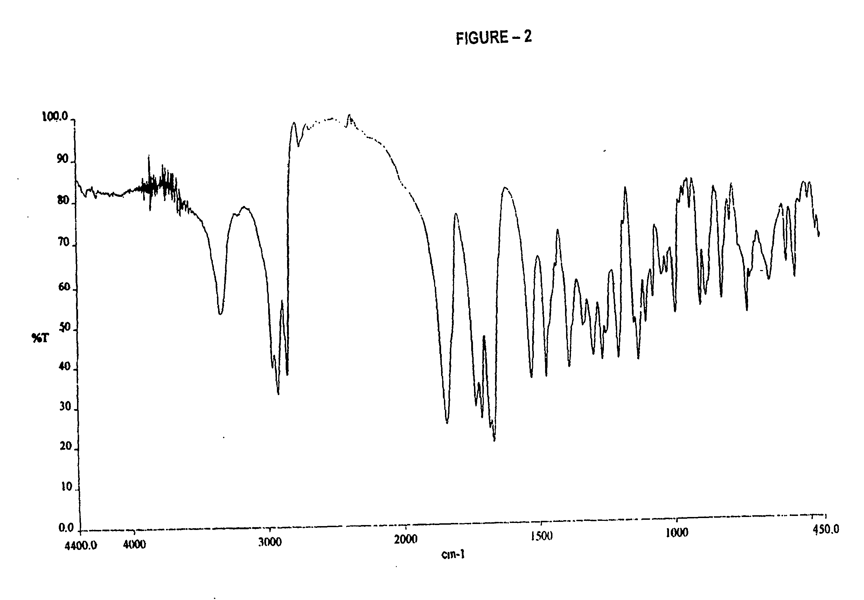

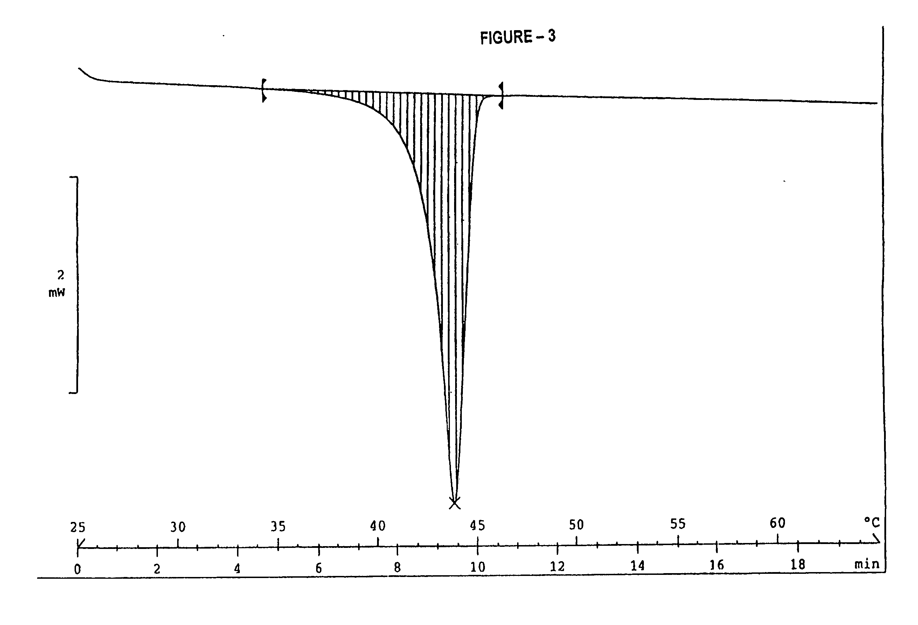

The process may produce the crystalline Form I of the orlistat having the X-ray diffraction pattern of Figure 1, the infrared spectrum of Figure 2, and the differential scanning calorimetry plot of Figure 3.

Pharmaceutical compositions that include a therapeutically acceptable amount of Form I of orlistat; prepared according to this invention, comprising one or more pharmaceutically acceptable carriers, excipients or diluents, can be prepared by using conventional formulation techniques. Such pharmaceutical compositions can be used for treating or preventing obesity and hyperlipaemia in a warm-blooded animal by providing a pharmaceutical composition to the warm-blooded animal that includes Form I of orlistat.

The details of one or more embodiments are set forth in the description below. Other features and aspects of the disclosure will be apparent from the description and claims.

### Description of the Drawings

Figure 1 is X-ray powder diffraction (XRD) pattern of Form I of orlistat.
Figure 2 is an infrared spectrum of Form I of orlistat.
Figure 3 is differential scanning calorimetry (DSC) plot of Form I of orlistat.

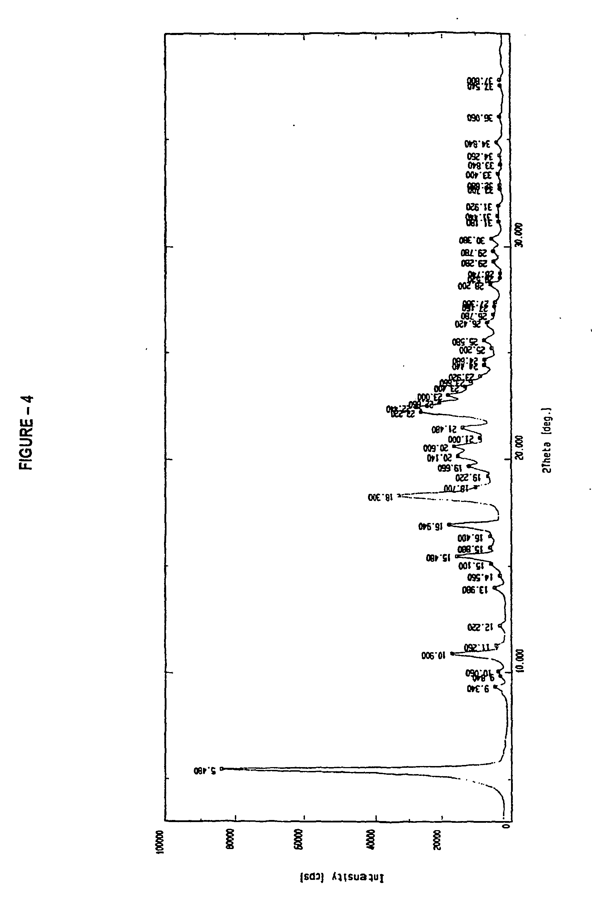

Figure 4 is X-ray powder diffraction (XRD) pattern of Form II of orlistat.

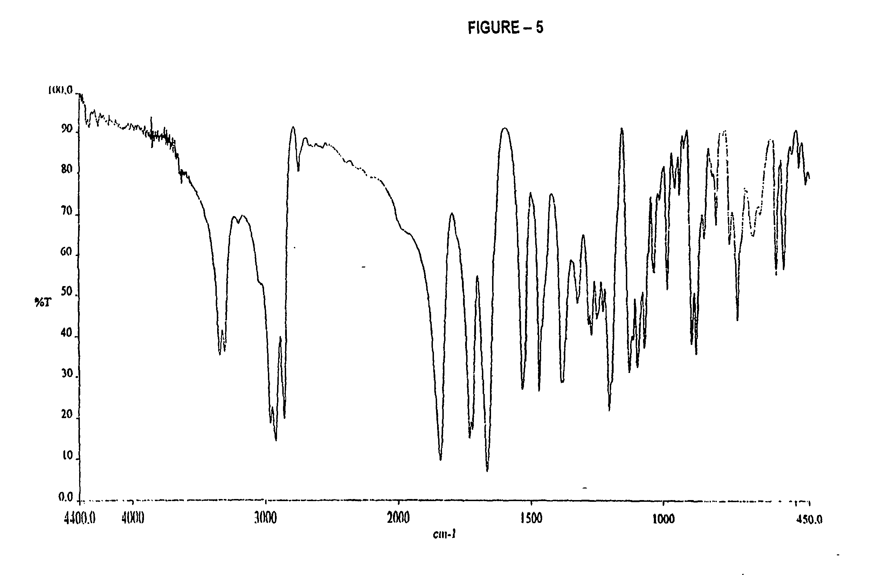

Figure 5 is an infrared spectrum of Form II of orlistat.

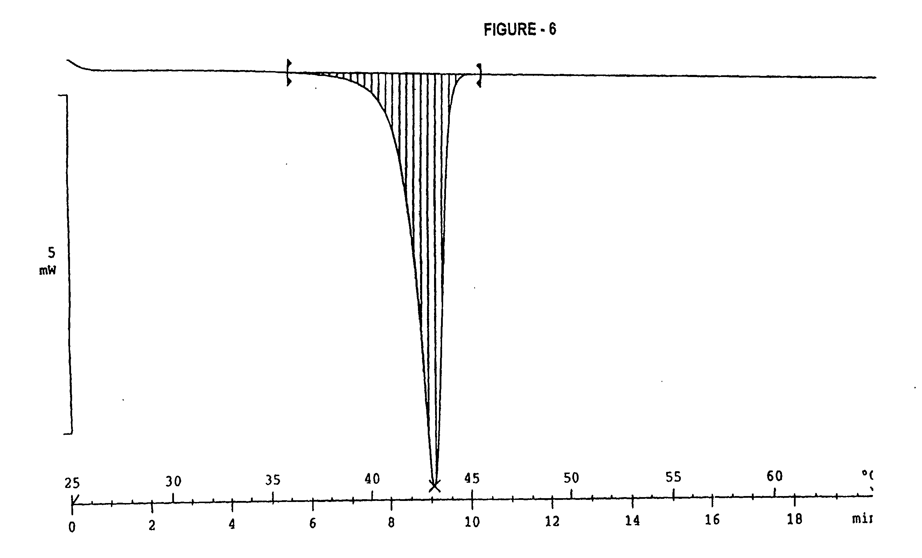

Figure 6 is differential scanning calorimetry (DSC) plot of Form II of orlistat.

### Detailed Description of the Invention

The inventors have also developed a process for the preparation of crystalline Form I of orlistat. The process involves obtaining a melt of orlistat and drying the melt to get the Form I of orlistat.

In general, the melt of orlistat may be obtained by heating any of the various forms known in the art including Form II, amorphous or waxy form of orlistat. It may be heated at a temperature from about 25°C to about 80°C, or at a temperature from about 30°C to about 60°C. In particular, it may be heated at a temperature from about 40°C to about 50°C. Alternatively, such a melt may also be obtained by dissolving orlistat in a suitable solvent and removing the solvent by any of the conventional techniques including, for example distillation, distillation under vacuum and evaporation.

The crystalline Form II of orlistat can be prepared by preparing a solution of orlistat in one or more ethers; and isolating the crystalline Form II from the solution thereof by the removal of the ether. In general, this solution of orlistat may be obtained by dissolving any of the various forms known in the art including Form I, amorphous or waxy form of orlistat in suitable ether. Alternatively, such a solution may be obtained directly from a reaction in which orlistat is formed. The ether containing orlistat may be heated to obtain a solution.

The ether may be removed from the solution by a technique which includes, for example, distillation, distillation under vacuum, evaporation, filtration, filtration under vacuum, decantation, and centrifugation. The solution may be cooled before removal of the ether to obtain better yields of the Form II of orlistat. Suitable ethers include diethyl ether, diisopropyl ether, tert.-butyl-methyl ether, and tetrahydrofuran. Mixtures of all of these ethers are also contemplated. In general, the amount of ether should be sufficient to dissolve the orlistat to get a solution. In particular, the volume of diisopropyl ether to dissolve orlistat may be from about 2 to 10 times the weight of orlistat.

The Form II orlisat obtained by removal of the ether may be further or additionally dried to obtain the desired moisture values. For example, the product may be further or additionally dried in a tray drier, dried under vacuum and/or in a Fluid Bed Drier.

In general, the starting orlistat may be prepared using the reactions and techniques known in the art including those described in U.S. Patent Nos. 4,598,089; 4,983,746; 6,156,911; 5,412,110; 2002110873; Helv. Chim. Acta. 1987, 70, 1412; J. Org.Chem., 1988, 53, 1218; 1993, 58, 7768; 1991, 56, 4714; J. Chem, Soc. Perkin. Trans, I, 1998, 17, 2679; Tetrahedron letters, 1989, 30, 1833; and Chem. Commun. 1999, 17, 1743.

The melted orlistat may be dried under reduced pressure to get the Form I of orlistat. In general, the melt may be cooled before drying to get a good crystalline solid.

The resulting crystalline Form I of orlistat may be formulated into ordinary dosage forms such as, for example, tablets, capsules, pills, solutions, etc. In these cases, the medicaments can be prepared by conventional methods with conventional pharmaceutical excipients.

The compositions include dosage forms suitable for oral, buccal, rectal, and parenteral (including subcutaneous, intramnuscular, and ophthalmic) administration. The oral dosage forms may include solid dosage forms, like powder, tablets, capsules, suppositories, sachets, troches and lozenges as well as liquid suspensions, emulsions, pastes and elixirs. Parenteral dosage forms may include intravenous infusions, sterile solutions for intramuscular, subcutaneous or intravenous administration, dry powders to be reconstituted with sterile water for parenteral administration, and the like.

The orlistat of crystalline Form I can be administered for the treatment and prevention of obesity and hyperlaemia, in a warm-blooded animal. For the purpose of this disclosure, a warm-blooded animal is a member of the animal kingdom possessed of a homeostatic mechanism and includes mammals and birds.

The present invention is further illustrated by the following examples which are provided to be exemplary of the invention.

### Example 1: Preparation of orlistat Form II

Orlistat Form I (2.0 g) was dissolved in diisopropyl ether (10.0 ml) and stirred at room temperature to get a clear solution. The reaction mixture was cooled to 0 to-5°C and the product was precipitated slowly with stirring. The product was filtered and washed with diisopropyl ether. It was then dried at room temperature under vacuum to obtain white crystals of orlistat.
Yield: 1.7 g

XRD, IR spectra and DSC spectra were similar to those shown in Figure 4, 5 and 6, respectively.

### Example 2: Preparation of orlistat Form I

2.0 g of orlistat form II was taken and the temperature was raised to 42°C using a water bath to form a melted syrup. The syrupy material was dried under reduced pressure to obtain white crystalline orlistat.

XRD, IR spectra and DSC spectra were similar to those shown in Figure 1, 2 and 3, respectively.

## Claims

1. A process for the preparation of crystalline Form I of orlistat wherein the orlistat Form I is a crystalline form of orlistat obtainable by recrystallization of orlistat with hydrocarbons as disclosed in U.S. Patent No. 6,156,911; *Tetrahedron letters*, 1989, 30, 1833; *J. Org. Chem*., 1991, 56, 4714; and Helv. *Chim. Acta*. 1987, 70, 1412, the process comprising:
a. obtaining a melt of orlistat; and
b. drying the melt to get the Form I of orlistat,

2. The process of claim 1, wherein the melt is obtained by heating any known form of orlistat, including amorphous orlistat.

3. The process of claim 2, wherein crystalline Form II of orlistat is used to make the melt.

4. The process of claim 3, wherein said crystalline Form II of orlistat is prepared by the process comprising:
a. preparing a solution of orlistat in one or more ethers; and b. isolating the orlistat in the crystalline Form II from the solution thereof by the removal of the ether.

5. The process of claim 4, wherein the ether comprises one or more of diethyl ether, diisopropyl ether, tert.-butyl-methyl ether, and tetrahydrofuran.

6. The process of claim 2, wherein the heating is performed at a temperature from about 25°C to about 80°C.

7. The process of claim 6, wherein the heating temperature is from about 40°C to about 50°C.

8. The process of claim 1, wherein the drying is performed under reduced pressure.

9. The process of claim 1, further comprising cooling before drying the melt.

10. The process of claim 1, further comprising forming the product obtained into a finished dosage form.

11. The process of any preceding claim wherein the orlistat Form I obtained is formulated into a pharmaceutical composition comprising the orlistat Form I and one or more pharmaceutically acceptable carriers, excipients or diluents.

## Patentansprüche

1. Verfahren zur Herstellung einer kristallinen Form I von Orlistat, wobei die Orlistat-Form I eine kristalline Form von Orlistat ist, welche erhältlich ist durch Rekristallisation von Orlistat mit Kohlenwasserstoffen, wie in US-Patent Nr. 6,156,911; Tetrahedron letters, 1989, 30, 1833; J. Org. Chem., 1991, 56, 4714; und Helv. Chim. Acta. 1987, 70, 1412; beschrieben ist, wobei das Verfahren umfasst:
a. Erhalten einer Schmelze von Orlistat; und
b. Trocknen der Schmelze, um die Form I von Orlistat zu erhalten.

2. Verfahren nach Anspruch 1, wobei die Schmelze erhalten wird durch Erwärmen irgendeiner bekannten Form von Orlistat, einschließlich amorphem Orlistat.

3. Verfahren nach Anspruch 2, wobei eine kristalline Form II von Orlistat verwendet wird, um die Schmelze herzustellen.

4. Verfahren nach Anspruch 3, wobei die kristalline Form II von Orlistat hergestellt wird durch ein Verfahren umfassend:
a. Herstellung einer Lösung von Orlistat in einem oder mehreren Ethern; und
b. Isolierung von Orlistat in der kristallinen Form II aus der Lösung desselben durch Entfernung des Ethers.

5. Verfahren nach Anspruch 4, wobei der Ether ein oder mehrere aus der Gruppe Diethylether, Diisopropylether, tert.-Butylmethylether und Tetrahydrofuran enthält.

6. Verfahren nach Anspruch 2, wobei die Erwärmung bei einer Temperatur von ungefähr 25°C bis ungefähr 80°C durchgeführt wird.

7. Verfahren nach Anspruch 6, wobei die Erwärmungstemperatur in dem Bereich von ungefähr 40°C bis ungefähr 50°C liegt.

8. Verfahren nach Anspruch 1, wobei die Trocknung unter reduziertem Druck durchgeführt wird.

9. Verfahren nach Anspruch 1, wobei vor der Trocknung der Schmelze eine Kühlung vorgenommen wird.

10. Verfahren nach Anspruch 1, wobei weiterhin umfasst ist, dass das erhaltene Produkt in eine endgültige Dosierungsform überführt wird.

11. Verfahren nach einem der vorhergehenden Ansprüche, wobei die erhaltene Orlistat-Form I in eine pharmazeutische Zusammensetzung enthaltend die Orlistat-Form I und ein oder mehrere pharmazeutisch akzeptable Träger, Exzipienten oder Verdünnungsmittel formuliert wird.

## Revendications

1. Procédé de préparation de la Forme I cristalline d'orlistat, la Forme I d'orlistat étant une forme cristalline d'orlistat pouvant être obtenue par recristallisation d'orlistat avec des hydrocarbures comme cela est décrit dans le brevet U.S. n° 6 156 911 ; *Tetrahedron letters,* 1989, 30, 1833 ; *J. Org. Chem*., 1991, 56, 4714 ; et Helv. *Chim. Acta*. 1987, 70, 1412, le procédé consistant à :
a. obtenir de l'orlistat fondu ; et
b. sécher le produit fondu pour obtenir la Forme I d'orlistat.

2. Procédé selon la revendication 1, dans lequel le produit fondu est obtenu en chauffant n'importe quelle forme connue d'orlistat, y compris l'orlistat amorphe.

3. Procédé selon la revendication 2, dans lequel une Forme II cristalline d'orlistat est utilisée pour faire le produit fondu.

4. Procédé selon la revendication 3, dans lequel ladite Forme II cristalline d'orlistat est préparée par le procédé consistant à :
a. préparer une solution d'orlistat dans un ou plusieurs éthers ; et
b. isoler l'orlistat dans la Forme II cristalline à partir de sa solution par élimination de l'éther.

5. Procédé selon la revendication 4, dans lequel l'éther comprend un ou plusieurs éléments parmi le diéthyléther, le diisopropyléther, le tert-butyl-méthyléther et le tétrahydrofurane.

6. Procédé selon la revendication 2, dans lequel le chauffage est effectué à une température allant d'environ 25°C à environ 80°C.

7. Procédé selon la revendication 6, dans lequel la température de chauffage est d'environ 40°C à environ 50°C.

8. Procédé selon la revendication 1, dans lequel le séchage est effectué sous pression réduite.

9. Procédé selon la revendication 1, comprenant en outre un refroidissement avant le séchage du produit fondu.

10. Procédé selon la revendication 1, consistant en outre à former le produit obtenu en une forme posologique finie.

11. Procédé selon l'une quelconque des revendications précédentes dans lequel la Forme I d'orlistat obtenue est formulée en une composition pharmaceutique comprenant la Forme I d'orlistat et un ou plusieurs supports, excipients ou diluants pharmaceutiquement acceptables.
